**Europäisches Patentamt**

**European Patent Office**

**Office européen des brevets**

(19)

(11) Veröffentlichungsnummer : **0 122 624**
**B1**

(12) **EUROPÄISCHE PATENTSCHRIFT**

(45) Veröffentlichungstag der Patentschrift :
07.09.88

(51) Int. Cl.⁴ : **A 61 K 49/00**

(21) Anmeldenummer : 84104210.4

(22) Anmeldetag : 13.04.84

(54) **Mikropartikel und Gasbläschen enthaltendes Ultraschallkontrastmittel.**

(30) Priorität : 15.04.83 DE 3313946

(43) Veröffentlichungstag der Anmeldung :
24.10.84 Patentblatt 84/43

(45) Bekanntmachung des Hinweises auf die Patenterteilung : 07.09.88 Patentblatt 88/36

(84) Benannte Vertragsstaaten :
AT BE CH DE FR GB IT LI LU NL SE

(56) Entgegenhaltungen :
EP-A- 0 052 575
EP-A- 0 077 752
US-A- 4 265 251

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Hilmann, Jürgen**
**Ugandastrasse 9**
**D-1000 Berlin 65 (DE)**
Erfinder : **Lange, Lothar, Dr.**
**Beskidenstrasse 24**
**D-1000 Berlin 38 (DE)**
Erfinder : **Zimmermann, Ingfried, Dr.**
**Gollanczstrasse 28c**
**D-1000 Berlin 28 (DE)**

EP 0 122 624 B1

## Beschreibung

Die Erfindung betrifft Mikropartikel und Gasbläschen enthaltende Kontrastmittel für die Ultraschall-Diagnostik, dadurch gekennzeichnet, daß es Mikropartikel einer festen, grenzflächenaktiven Substanz, gegebenenfalls in Kombination mit Mikropartikel eines nicht grenzflächenaktiven Feststoffes in einem flüssigen Träger enthält.

Die Untersuchung von Organen mit Ultraschall (Sonographie) ist eine seit einigen Jahren gut eingeführte und praktizierte diagnostische Methode. Ultraschallwellen im Megahertz-Bereich (oberhalb 2 Mega-Hertz mit Wellenlängen zwischen 1 und 0,2 mm) werden an Grenzflächen von unterschiedlichen Gewebearten reflektiert. Die dadurch entstehenden Echos werden verstärkt und sichtbar gemacht. Von besonderer Bedeutung ist dabei die Untersuchung des Herzens mit dieser Methode, die Echokardiographie genannt wird (Haft, J.I. et al. : Clinical echocardiography, Futura, Mount Kisco, New York 1978 ; Köhler, E. Klinische Echokardiographie, Enke, Stuttgart 1979 ; Stefan, G. et al. : Echokardiographie, Thieme, Stuttgart-New York 1981 ; G. Biamino, L. Lange : Echokardiographie, Hoechst AG, 1983.

Da Flüssigkeiten — auch das Blut — nur dann Ultraschall-Kontrast liefern, wenn Dichte-Unterschiede zur Umgebung bestehen, wurde nach Möglichkeiten gesucht, das Blut und seine Strömung für eine Ultraschall-Untersuchung sichtbar zu machen, was durch die Zugabe von feinsten Gasbläschen auch möglich ist.

Aus der Literatur sind mehrere Methoden zur Herstellung und Stabilisierung der Gasbläschen bekannt. Sie lassen sich beispielsweise vor der Injektion in den Blutstrom durch heftiges Schütteln oder Rühren von Lösungen wie Salzlösungen, Farbstofflösungen oder von vorher entnommenem Blut erzeugen.

Obwohl man dadurch eine Ultraschall-Kontrastgebung erreicht, sind diese Methoden mit schwerwiegenden Nachteilen verbunden, die sich in schlechter Reproduzierbarkeit, stark schwankender Größe der Gasbläschen und — bedingt durch einen Anteil an sichtbaren großen Bläschen — einem gewissen Embolie-Risiko äußern. Diese Nachteile wurden durch andere Herstellungsverfahren teilweise behoben, wie beispielsweise durch das US-Patent 3, 640, 271, in dem Bläschen mit reproduzierbarer Größe durch Filtration oder durch die Anwendung einer unter Gleichstrom stehenden Elektrodenvorrichtung erzeugt werden. Dem Vorteil in der Möglichkeit, Gasbläschen mit reproduzierbarer Größe herstellen zu können, steht der erhebliche technische Aufwand als Nachteil gegenüber.

In dem US-Patent 4, 276, 885 wird die Herstellung von Gasbläschen mit definierter Größe beschrieben, die mit einer vor dem Zusammenfließen schützenden Gelatine-Hülle umgeben sind. Die Aufbewahrung der fertigen Bläschen kann nur im « eingefrorenen » Zustand erfolgen, beispielsweise durch Aufbewahren bei Kühlschranktemperatur, wobei sie zur Verwendung wieder auf Körpertemperatur gebracht werden müssen.

In dem US-Patent 4, 265, 251 wird die Herstellung und Verwendung von Gasbläschen mit einer festen Hülle aus Sacchariden beschrieben, die mit einem unter Druck stehenden Gas gefüllt sein können. Stehen sie unter Normaldruck, so können sie als Ultraschallkontrastmittel eingesetzt werden ; bei Verwendung mit erhöhtem Innendruck dienen sie der Blutdruckmessung. Obwohl hierbei die Aufbewahrung der festen Gasbläschen kein Problem darstellt, ist der technische Aufwand bei der Herstellung ein erheblicher Kostenfaktor.

Die Risiken der nach dem Stand der Technik zur Verfügung stehenden Kontrastmittel werden durch zwei Faktoren hervorgerufen : Größe und Anzahl sowohl der Feststoffpartikel als auch der Gasbläschen.

Der bisher geschilderte Stand der Technik gestattet die Herstellung von Ultraschallkontrastmitteln, die stets nur einige der geforderten Eigenschaften besitzen :

1. Ausschalten des Embolierisikos
   Gasbläschen (Größe und Anzahl)
   Feststoffpartikel (Größe und Anzahl)
2. Reproduzierbarkeit
3. genügend lange Stabilität
4. Lungengängigkeit, z. B. um Ultraschall-Kontrastierung des linken Herzteiles zu erhalten
5. Kapillargängigkeit
6. Sterilität und Pyrogenfreiheit der Zubereitung
7. leichte Herstellbarkeit mit vertretbarem Kostenaufwand
8. und problemlose Bevorratung.

In der europäischen Patentanmeldung mit der Veröffentlichungs-Nummer 52575 wird zwar die Herstellung von Gasbläschen beschrieben, die diese erforderlichen Eigenschaften besitzen sollen. Zu ihrer Herstellung werden Mikropartikel einer festen kristallinen Substanz, wie beispielsweise Galaktose, in einer Trägerflüssigkeit suspendiert, wobei das Gas, das an der Partikeloberfläche adsorbiert, in Hohlräumen zwischen den Partikel oder in interkristallinen Hohlräumen eingeschlossen ist, die Gasbläschen bildet. Die so entstandene Suspension von Gasbläschen und Mikropartikel wird innerhalb von 10 Minuten injiziert. Obwohl in der europäischen Patentschrift 52575 behauptet wird, daß die nach der beschriebenen Methode hergestellte Suspension geeignet ist, nach Injektion in eine periphere Vene sowohl auf der rechten Herzseite als auch nach Passage der Lunge in der linken Herz-Seite zu erscheinen und dort das Blut und dessen Strömung bei Ultraschall-Untersuchung sichtbar zu machen, hielt diese Behauptung einer Nachprüfung nicht stand. So wurde festgestellt, daß das nach der in der europäischen Anmeldung Nr. 52575 beschriebenen Methode hergestellte und in eine periphere Vene injizierte Kontrastmittel keine Ultraschall-Echos im linken Herzteil erzeugten.

Auch in der Ep-A-77752 wird die Herstellung von einer flüssigen Mischung zur Verwendung als Kontrastmittel beschrieben, das wiederum aus einer Mischung eines Tensides oder einer waßrigen Lösung des Tensides und einer waßrigen viskosen Trägerflüssigkeit besteht.

Es war die Aufgabe der vorliegenden Erfindung, ein Kontrastmittel für die Ultraschall-Diagnostik bereitzustellen, das in der Lage ist, nach intravenöser Applikation das Blut und dessen Strömungsverhältnisse nicht nur auf der rechten Seite des Herzens, sondern auch nach der Passage des Kapillarbettes der Lunge auf der linken Herzseite für Ultraschall sichtbar zu machen. Darüberhinaus soll es auch die Darstellung der Durchblutung anderer Organe wie Myocard, Leber, Milz und Niere gestatten.

Die neuen erfindungsgemäßen Mittel gemäß der Ansprüche 1 bis 14 besitzen alle Eigenschaften, die von einem solchen Kontrastmittel erwartet werden und die auf Seite 3 aufgezählt wurden.

Es wurde überraschenderweise festgestellt, daß durch Suspendieren von Mikropartikel einer festen grenzflächenaktiven Substanz gegebenenfalls in Kombination mit Mikropartikel eines nicht grenzflächenaktiven Feststoffes in einer Trägerflüssigkeit ein Ultraschall-Kontrastmittel erhalten wird, das nach Injektion in eine periphere Vene auch vom Blut in der arteriellen linken Herzseite reproduzierbare Ultraschall-Aufnahme ermöglicht. Da mit dem erfindungsgemäßen Ultraschall-Kontrastmittel nach intravenöser Gabe die linke Herzseite erreicht werden kann, sind so auch Ultraschall-Kontraste von anderen von der Aorta aus mit Blut versorgten Organen nach venöser Applikation möglich, wie Myokard, Leber, Milz, Niere u.a.m. Es versteht sich von selbst, daß das erfindungsgemäße Ultraschall-Kontrastmittel auch für Rechtsherz-Kontraste und für alle übrigen Anwendungen als Ultraschall-Kontrastmittel geeignet ist.

Als grenzflächenaktive Substanz für die Herstellung von Mikropartikel sind alle Stoffe geeignet, die in den angewandten Mengen physiologisch verträglich sind, d. h. die eine geringe Toxizität besitzen und/oder biologisch abbaubar sind und deren Schmelzpunkt größer als Raumtemperatur ist. Insbesondere geeignet sind Lecithine, Lecithinfraktionen und deren Abwandlungsprodukte, Polyoxyethylenfettsäureester wie Polyoxyethylenfettalkoholäther, polyoxyethylierte Sorbitanfettsäureester, Glycerin-poly-ethylenglykoloxystearat, Glycerinpolyethylenglykolrhizinoleat, ethoxylierte Sojasterine, ethoxylierte Rizinusöle und deren hydrierte Derivate, Cholesterol, Polyoxyethylenfettsäurestearate und Polyoxyethylenpolyoxypropylen-Polymere mit dem Molgewicht von 6800-8975, 13300 und 16250, Saccharoseester wie Zuckerester, beispielsweise Saccharosedipalmitat und Saccharosemonolaurat oder Saccharoseglyceride sowie Xyloglyceride, gesättigte oder ungesättigte $(C_4-C_{20})$-Fettalkohole oder $(C_4-C_{20})$-Fettsäuren oder deren Metallsalze, Polyoxyäthylenfettsäureester, Mono-, Di- und Triglyceride, Sorbitanfettsäureester, Fettsäureester der Saccharose oder Fettsäureester wie Butylstearat und Ascorbylpalmitat, wobei Calciumstearat, die Saccharoseester der Laurinsäure, der Stearinsäure und der Palmitinsäure sowie Ascorbylpalmitat bevorzugt sind.

Als grenzflächenaktive Substanzen sind solche besonders gut geeignet, die in der Trägerflüssigkeit relativ schwer löslich und im Blut relativ leicht löslich sind. Durch diesen Sprung im Auflöseverhalten der grenzflächenaktiven Substanzen kann die Auflösung des festen Materials, das reich an eingeschlossener Luft ist, gesteuert werden.

Die grenzflächenaktive Substanz wird in einer Konzentration von 0,01 bis 5 Gewichtsprozent, vorzugsweise von 0,04 bis 0,5 Gewichtsprozent verwendet.

Falls erwünscht, können die Mikropartikel der grenzflächenaktiven Substanz mit Mikropartikel eines physiologisch verträglichen kristallinen Feststoffes kombiniert werden. Man kann dafür organische und anorganische Stoffe verwenden, zum Beispiel Salze wie Natriumchlorid, Natriumcitrat, Natriumacetat oder Natriumtartrat, Monosaccharide wie Glucose, Fructose oder Galaktose, Disaccharide wie Saccharose, Lactose oder Maltose, Pentosen wie Arabinose, Xylose oder Ribose oder Cyclodextrine wie α-, β- oder γ-Cyclodextrin, wobei Galactose, Lactose und α-Cyclodextrin bevorzugt sind. Sie sind in einer Konzentration von 5 bis 50 Gewichtsprozent, vorzugsweise von 9 bis 40 Gewichtsprozent, im erfindungsgemäßen Mittel enthalten.

Zur Herstellung der Mikropartikel werden die dafür vorgesehenen Substanzen unter sterilen Bedingungen rekristallisiert. Anschließend werden sie unter sterilen Bedingungen zerkleinert, z. B. durch Vermahlung in einer Luftstrahlmühle, bis die gewünschte Partikelgröße erreicht ist. Erhalten wird eine Partikelgröße von < 10 µm, vorzugsweise < 8 µm, insbesondere 1-3 µm. Die Partikelgröße wird in geeigneten Meßgeräten bestimmt. Die erzeugten Mikropartikel bestehen entweder aus der zerkleinerten grenzflächenaktiven Substanz allein oder aus einer Mischung der Mikropartikel von grenzflächenaktiver Substanz und nicht grenzflächenaktivem Feststoff. In diesem Fall beträgt das Gewichtsverhältnis von fester grenzflächenaktiver Substanz zu nicht grenzflächenaktivem Feststoff 0,01 bis 5 zu 100.

Sowohl die durch das Zerkleinerungsverfahren erreichte Größe der Mikropartikel als auch die Größe der im erfindungsgemäßen Kontrastmittel enthaltenen Gasbläschen gewährleistet eine gefahrlose Passage des Kapillarsystems und des Lungenkapillarbettes und schließt das Entstehen von Embolien aus.

Die für die Kontrastgebung benötigten Gasbläschen werden teilweise durch die suspendierten Mikropartikel transportiert, an der Oberfläche der Mikropartikel absorbiert, in den Hohlräumen zwischen den Mikropartikel oder interkristallin eingeschlossen.

Das von den Mikropartikel transportierte Gasvolumen in Form von Gasbläschen beträgt 0,02 bis 0,6 ml pro Gramm Mikropartikel.

Die Trägerflüssigkeit hat neben der Transportfunktion die Aufgabe, die aus Mikropartikel und Gasbläschen bestehende Suspension zu stabilisieren, z. B. das Sedimentieren der Mikropartikel und das Zusammenfließen der Gasbläschen zu verhindern bzw. den Lösevorgang der Mikropartikel zu verzögern.

Als flüssiger Träger kommen in Frage Wasser, wässrige Lösungen eines oder mehrerer anorganischer Salze wie physiologische Kochsalzlösung und Pufferlösungen, wässrige Lösungen von Mono- oder Disacchariden wie Galactose, Glucose oder Lactose, ein- oder mehrwertige Alkohole, soweit sie physiologisch verträglich sind wie Äthanol, Propanol, Isopropylalkohol, Polyethylenglykol, Ethylenglykol, Glycerin, Propylenglykol, Propylenglycolmethylester oder deren wässrige Lösungen.

Bevorzugt sind Wasser und physiologische Elektrolytlösungen wie physiologische Kochsalzlösung sowie wässrige Lösungen von Galactose und Lactose. Werden Lösungen verwendet, beträgt die Konzentration des gelösten Stoffes 0,1 bis 30 Gewichtsprozent, vorzugsweise 0,5 bis 25 Gewichtsprozent, insbesondere verwendet man 0,9 %ige wässrige Kochsalzlösung oder 20 %ige wässrige Galactose-Lösung.

Die Erfindung betrifft auch ein Verfahren zur Herstellung des erfindungsgemäßen Mittels gemäß Anspruch 16.

Zur Herstellung des gebrauchsfertigen Ultraschall-Kontrastmittels gibt man die sterile Trägerflüssigkeit zu der in Form von Mikropartikel vorliegenden sterilen festen grenzflächenaktiven Substanz, die gegebenenfalls mit einem sterilen nicht-grenzflächenaktiven Stoff kombiniert ist und schüttelt diese Mischung, bis sich eine homogene Suspension gebildet hat, wofür ca. 5 bis 10 sec. erforderlich sind. Die entstandene Suspension wird sofort nach ihrer Herstellung, spätestens jedoch bis 5 Minuten danach als Bolus in eine periphere Vene oder einen schon vorhandenen Katheder injiziert, wobei von 0,01 ml bis 1 ml/kg Körpergewicht appliziert werden.

Aus Gründen der Zweckmäßigkeit werden die zur Herstellung des erfindungsgemäßen Mittels benötigten Komponenten wie Trägerflüssigkeit (A) und Mikropartikel der grenzflächenaktiven Substanz, gegebenenfalls kombiniert mit Mikropartikel des nicht-grenzflächenaktiven Feststoffes (B) in der für eine Untersuchung erforderlichen Menge steril in zwei getrennten Gefäßen aufbewahrt. Beide Gefäße haben Verschlüsse, die Entnahme und Zugabe mittels Injektionsspritze unter sterilen Bedingungen ermöglichen (Vials). Die Größe von Gefäß B muß so beschaffen sein, daß der Inhalt von Gefäß A mittels Injektionsspritze nach B überführt werden kann und die vereinigten Komponenten geschüttelt werden können. Die Erfindung betrifft deshalb auch ein Kit gemäß Anspruch 15.

Die Durchführung einer echokardiographischen Untersuchung an einem 10 kg schweren Pavian soll die Verwendung des erfindungsgemäßen Kontrastmittels demonstrieren :

5 ml Trägerflüssigkeit (hergestellt nach Beispiel 1 A) werden aus einem Vial mit einer Injektionsspritze entnommen und zu 2 g Mikropartikel (hergestellt nach Beispiel 1 B), die sich in einem zweiten Vial befinden gegeben und etwa 5-10 sec. geschüttelt, bis sich eine homogene Suspension gebildet hat. Man injiziert 2 ml dieser Suspension in eine periphere Vene (V. jugularis, brachialis oder saphena) über einen 3-Wege-Hahn mit einer Infusionsgeschwindigkeit von mindestens 1 ml/sec., besser mit 2-3 ml/sec. An die Kontrastmittelinjektion schließt sich mit derselben Geschwindigkeit sofort die Injektion von 10 ml physiologischer Kochsalzlösung an, damit der Kontrastmittel-Bolus so weitgehend wie möglich bis zum Erreichen des rechten Herzteils erhalten bleibt. Vor, während und nach der Injektion wird ein handelsüblicher Schallkopf für die Echokardiographie an den Thorax des Versuchstieres gehalten, so daß ein typischer Querschnitt durch das rechte und linke Herz erhalten wird. Diese Versuchsanordnung entspricht dem Stand der Technik und ist dem Fachmann bekannt.

Erreicht das Ultraschallkontrastmittel das rechte Herz, kann man im 2-D-Echobild oder im M-mode-Echo-Bild verfolgen, wie das durch das Kontrastmittel markierte Blut zunächst die Höhe des rechten Vorhofes, dann die des rechten Ventrikels und der Pulmonalarterie erreicht, wobei für ca. 10 sec. eine homogene Füllung auftritt. Während die Höhlen des rechten Herzens im Ultraschallbild wieder leer werden, erscheint das mit Kontrastmittel markierte Blut nach der Lungenpassage in den Pulmonalvenen wieder, füllt den linken Vorhof, den linken Ventrikel und die Aorta homogen, wobei der Kontrast 2 bis 3 mal länger anhält als auf der rechten Herzseite. Neben der Darstellung des Blutflusses durch die Höhlen des linken Herzens kommt es auch zu einer Darstellung des Myokards, die die Durchblutung wiederspiegelt.

Die Verwendung des erfindungsgemäßen Ultraschall-Kontrastmittels ist aber nicht beschränkt auf die Sichtbarmachung des Blutstroms im arteriellen Teil des Herzens nach venöser Applikation sondern es wird mit ausgezeichneten Erfolg auch bei der Untersuchung des rechten Herzens und anderer Organe als Kontrastmittel verwendet.

Beispiel 1

A) Herstellung der Trägerflüssigkeit :

80 g Galactose werden im Wasser für Injektionszwecke gelöst bis zu einem Volumen von 400 ml aufgefüllt durch ein 0,2 μm-Filter gedrückt, jeweils 4 ml dieses Filtrats in 5 ml-Vials abgefüllt und 20 Minuten bei 120 °C sterilisiert.

B) Herstellung der Mikropartikel :

Unter sterilen Bedingungen werden 198 g Galactose mit 2 g Magnesiumstearat durch homöopathische Anreibung intensiv vermischt, durch ein 0,8 mm-Sieb gegeben, lose gemischt und mit einer Luftstrahlmühle vermahlen, bis folgende Verteilung der Partikelgröße erreicht ist :

Median 1,9 μm
99 % < 6 μm
90 % < 3 μm.

Die Bestimmung der Partikelgröße und deren Verteilung erfolgt im Partikel-Meßgerät nach Suspendierung im wasserfreiem Isopropanol. Die Mikropartikel werden zu je 2 g in 5 ml-Vials abgefüllt.

C) Zur Herstellung von 5 ml des gebrauchsfertigen Ultraschall-Kontrastmittels wird der Inhalt eines Vials mit Trägerflüssigkeit (20 % Galactose-lösung in Wasser, A) mittels einer Injektionsspritze in das Vial mit Mikropartikeln (B) gegeben und bis zum Entstehen einer homogenen Suspension geschüttelt (5 bis 10 Sekunden).

Beispiel 2

A) Herstellung der Trägerflüssigkeit :

Es wird Wasser für Injektionszwecke verwendet, jeweils 4 ml in 5 ml-Vials abgefüllt und diese 20 Minuten bei 120 °C sterilisiert.

B) Herstellung der Mikropartikel :

Unter sterilen Bedingungen werden 198 g Galactose mit 2 g Ascorbylpalmitat durch homöopathische Anreibung intensiv vermischt und weiter verarbeitet, wie bei Beispiel 1 unter B) beschrieben, wobei sich folgende Verteilung der Partikelgröße ergibt :

Median 1,9 μm
100 % < 6 μm
90 % < 3 μm.

Die Bestimmung der Partikelgröße erfolgt wie in Beispiel 1 unter B) beschrieben.

Die Mikropartikel werden zu je 1200 mg in 5 ml-Vials abgefüllt.

C) Zur Herstellung von 4,5 ml des gebrauchsfertigen Ultraschall-Kontrastmittels wird der Inhalt eines Vials mit Trägerflüssigkeit (Wasser, A) mittels einer Injektionsspritze in ein Vial mit Mikropartikel, (B) gegeben und bis zum Entstehen einer homogenen Suspension (5 bis 10 Sekunden) geschüttelt.

Beispiel 3

A) Herstellung der Trägerflüssigkeit :

Man löst 4,5 g Natriumchlorid in Wasser bis zum Volumen von 500 ml, drückt die Lösung durch ein 0,2 μm-Filter, füllt jeweils 4 ml dieser Lösung in 5 ml-Vials und sterilisiert 20 Minuten bei 120 °C.

B) Herstellung der Mikropartikel :

Unter sterilen Bedingungen werden 198 g Lactose anhydrous (< 0,3 mm) mit 2 g Ascorbylpalmitat durch homöopathische Anreibung intensiv vermischt und die Mischung weiter verarbeitet wie in Beispiel 1 unter B) beschrieben, wobei sich folgende Verteilung der Partikelgröße ergibt :

Median 2,8 μm

100 % < 48 μm
99 % < 12 μm.

Die Bestimmung der Partikelgröße erfolgt wie in Beispiel 1 unter B) beschrieben.

Die Mikropartikel werden zu je 1,6 g in 5 ml-Vials abgefüllt.

C) Zur Herstellung von 5 ml des gebrauchsfertigen Ultraschall-Kontrastmittels wird der Inhalt eines Vials (0,9 % Natriumchloridlösung in Wasser, A) mittels einer Injektionsspritze in ein Vial mit Mikropartikel (B) gegeben und bis zum Entstehen einer homogenen Suspension (5 bis 10 Sekunden) geschüttelt.

Beispiel 4

A) Herstellung der Trägerflüssigkeit :

Wie in Beispiel 3 unter A) beschrieben stellt man 0,9 %ige wässrige Natriumchloridlösung her, füllt sie in 4 ml-Portionen in 5 ml-Vials ab und sterilisiert 20 Minuten bei 120 °C.

B) Herstellung der Mikropartikel :

Unter sterilen Bedingungen werden 199 g α-Cyclodextrin mit 1 g Ascorbylpalmitat durch homöopathische Anreibung intensiv vermischt und die Mischung weiterverarbeitet wie in Beispiel 1 unter B) beschrieben, wobei man Mikropartikel mit folgender Größenverteilung erhält :

Median 2 μm
99 % < 6 μm
90 % < 4 μm.

Die Bestimmung der Partikelgröße erfolgt wie in Beispiel 1 unter B) beschrieben.

Die Mikropartikel werden zu je 400 mg in 5 ml-Vials abgefüllt.

C) Zur Herstellung von 4 ml des gebrauchsfertigen Ultraschall-Kontrastmittels wird der Inhalt eines Vials (0,9 %ige wässrige Natriumchloridlösung, A) mittels einer Injektionsspritze in ein Vial mit Mikropartikel (B) gegeben und bis zum Entstehen einer homogenen Suspension (5 bis 10 Sekunden) geschüttelt.

Beispiel 5

A) Herstellung der Trägerflüssigkeit :

50 g Lactose werden in Wasser für Injektionszwecke gelöst, bis zu einem Volumen von 500 ml aufgefüllt, durch ein 0,2 μm-Filter gedrückt, zu jeweils 4 ml in 5 ml-Vials gefüllt und 20 Minuten bei 120 °C sterilisiert.

B) Herstellung der Mikropartikel :

Ascorbylpalmitat wird in Methanol gelöst, durch ein 0,2 μm-Filter steril filtriert, unter sterilen Bedingungen rekristallisiert, getrocknet und durch ein 0,8 mm-Sieb gegeben. Anschließend wird das sterile Ascorbylpalmitat unter sterilen Bedingungen mit einer Luftstrahlmühle vermahlen, bis folgende Größenverteilung der Partikel erreicht ist :

Medianwert 1,9 μm

99 % < 6 μm
90 % < 3 μm.

Die Bestimmung der Partikelgröße und deren Verteilung erfolgt im Partikel-Meßgerät nach Suspendierung in kalter wässriger 0,1 %iger Pluronic F68-Lösung. Die Mikropartikel werden zu je 40 mg in sterile 5 ml-Vials abgefüllt.

C) Zur Herstellung von 4 ml des gebrauchsfertigen Ultraschall-Kontrastmittels wird der Inhalt eines Vials mit Trägerflüssigkeit (10 %ige Lactose-Lösung, A) mittels einer Injektionsspritze in das Vial mit den Mikropartikel gegeben und bis zum Entstehen einer homogenen Suspension geschüttelt.

Beispiel 6

A) Herstellung der Trägerflüssigkeit
Man löst 4,5 g Natriumchlorid in Wasser bis zum Volumen von 500 ml, drückt die Lösung durch ein 0,2 μm-Filter, füllt jeweils 4 ml dieser Lösung in 5 ml-Vials und sterilisiert 20 Minuten bis 120 °C.

B) Herstellung der Mikropartikel
Unter sterilen Bedingungen wird eine sterilfiltrierte Lösung von 0,5 g Ascorbylpalmitat in 40 g Isopropanol auf 199,5 g sterile Galactosepartikel aufgezogen, bei 40 °C und 200 Torr das Isopropanol abgetrocknet und mit einer Luftstrahlmühle zerkleinert, bis folgende Größenverteilung der Partikel erreicht ist :

Medianwert 1,9 μm
99 % < 6 μm
90 % < 3 μm.

Die Bestimmung der Partikelgröße und deren Verteilung erfolgt in einem Partikelmeßgerät, z. B. nach Suspendierung in Isopropanol. Die Abpackung der Mikropartikel erfolgt in 5 ml-Vials zu jeweils 2 g.

C) Zur Herstellung von 5 ml des gebrauchsfertigen Ultraschall-Kontrastmittels wird der Inhalt eines Vials mit Trägerflüssigkeit (0,9 % Natriumchloridlösung in Wasser, A) mittels einer Injektionsspritze in das Vial mit Mikropartikel (B) gegeben und bis zum Entstehen einer homogenen Suspension geschüttelt (5 bis 10 Sekunden).

Beispiel 7

A) Herstellung der Trägerflüssigkeit
Man löst 4,5 g Natriumchlorid in Wasser, füllt bis zu einem Volumen von 500 ml auf, drückt die Lösung durch ein 0,2 μm-Filter, füllt jeweils 4 ml dieser Lösung in 5 ml-Vials und sterilisiert 20 Minuten bei 120 °C.

B) Herstellung der Mikropartikel
Unter sterilen Bedingungen werden 199,5 g Galactose mit 0,5 g Ascorbylpalmitat angerieben, intensiv gemischt, durch ein 0,8 mm-Sieb gegeben und mit einer Luftstrahlmühle zerkleinert, bis folgende Größenverteilung der Partikel erreicht ist :

Medianwert 1,9 μm
99 % < 6 μm
90 % < 3 μm.

Die Bestimmung der Partikelgröße und deren Verteilung erfolgt in einem Partikelmeßgerät, z. B. nach Suspendierung in Isopropanol. Die Abpackung der Mikropartikel erfolgt in 5 ml-Vials zu jeweils 2 g.

C) Zur Herstellung von 5 ml des gebrauchsfertigen Ultraschall-Kontrastmittels wird der Inhalt eines Vials mit Trägerflüssigkeit (0,9 % Natriumchloridlösung in Wasser, A) mittels einer Injektionsspritze in das Vial mit Mikropartikel (B) gegeben und bis zum Entstehen einer homogenen Suspension geschüttelt (5 bis 10 Sekunden).

Beispiel 8

A) Herstellung der Trägerflüssigkeit
Es wird Wasser für Injektionszwecke verwendet, jeweils zu 4 ml in 5 ml-Vials abgefüllt und diese 20 Minuten bei 120 °C sterilisiert.

B) Herstellung der Mikropartikel
Unter sterilen Bedingungen werden 0,5 g Saccharosemonopalmitat mit 199,5 g Galactose angerieben, intensiv gemischt, durch ein 0,8 mm-Sieb gegeben und mit einer Luftstrahlmühle vermahlen, bis folgende Größenverteilung der Partikel erreicht ist :

Medianwert 1,9 μm
min. 99 % < 6 μm
min. 90 % < 3 μm.

Die Bestimmung der Größe der Partikel und deren Verteilung erfolgt in einem Partikelmeßgerät, z. B. nach Suspendierung in Isopropanol. Die Abpackung der Mikropartikel erfolgt in 5 ml-Vials zu jeweils 2 g.

C) Zur Herstellung von 5 ml des gebrauchsfertigen Ultraschall-Kontrastmittels wird der Inhalt eines Vials mit Trägerflüssigkeit (steriles Wasser für Injektionszwecke, A) mittels einer Injektionsspritze in das Vial mit Mikropartikel (B) gegeben und bis zum Entstehen einer homogenen Suspension geschüttelt (5 bis 10 Sekunden).

Beispiel 9

A) Herstellung der Trägerflüssigkeit
Wasser für Injektionszwecke wird zu jeweils 4 ml in 5 ml-Vials abgefüllt und 20 Minuten bei 120 °C sterilisiert.

B) Herstellung der Mikropartikel
Unter sterilen Bedingungen wird eine sterilfiltrierte Lösung von 0,5 g Saccharosemonopalmitat in 40 g Isopropanol auf 199,5 g sterile Galactosepartikel aufgezogen, bei 40 °C und 200 Torr das Isopropanol abgetrocknet und mit einer Luftstrahlmühle vermahlen, bis folgende Größenverteilung der Partikel erreicht ist :

Medianwert 1,9 μm

min. 99 % < 6 μm
min. 90 % < 3 μm.

Die Bestimmung der Partikelgröße und deren Verteilung erfolgt in einem Partikelmeßgerät, z. B. nach Suspendierung in Isopropanol. Die Abpackung der Mikropartikel erfolgt in 5 ml-Vials zu jeweils 2 g.

C) Zur Herstellung von 5 ml des gebrauchsfertigen Ultraschallkontrastmittels wird der Inhalt eines Vials mit Trägerflüssigkeit (Wasser für Injektionszwecke, A) mittels einer Injektionsspritze in das Vial mit Mikropartikel (B) gegeben und bis zum Entstehen einer homogenen Suspension geschüttelt (5 bis 10 Sekunden).

Beispiel 10

A) Herstellung der Trägerflüssigkeit
Wasser für Injektionszwecke wird zu jeweils 4 ml in 5 ml-Vials abgefüllt und 20 Minuten bei 120 °C sterilisiert.
B) Herstellung der Mikropartikel
Unter sterilen Bedingungen wird eine sterilfiltrierte Lösung von 0,5 g Saccharosemonostearat in 40 g Isopropanol auf 199,5 g sterile Galactosepartikel aufgezogen, bei 40 °C und 200 Torr des Isopropanol abgetrocknet und mit einer Luftstrahlmühle vermahlen, bis folgende Größenverteilung der Partikel erreicht ist :

Medianwert 1,9 μm
min. 99 % < 6 μm
min. 90 % < 3 μm.

Die Bestimmung der Partikelgröße und deren Verteilung erfolgt in einem Partikelmeßgerät, z. B. nach Suspendierung in Isopropanol. Die Abpackung der Mikropartikel erfolgt in 5 ml-Vials zu jeweils 2 g.

C) Zur Herstellung von 5 ml des gebrauchsfertigen Ultraschall-Kontrastmittels wird der Inhalt eines Vials mit Trägerflüssigkeit (Wasser für Injektionszwecke, A) mittels einer Injektionsspritze in das Vial mit Mikropartikel (B) gegeben und bis zum Entstehen einer homogenen Suspension geschüttelt (5 bis 10 Sekunden).

Beispiel 11

A) Herstellung der Trägerflüssigkeit
Es wird Wasser für Injektionszwecke verwendet, jeweils zu 4 ml in 5 ml-Vials abgefüllt und diese 20 Minuten bei 120 °C sterilisiert.
B) Herstellung der Mikropartikel
Unter sterilen Bedingungen werden 0,5 g Saccharosemonostearat mit 199,5 Galactose angerieben, intensiv gemischt, durch ein 0,8 mm-Sieb gegeben und mit einer Luftstrahlmühle vermahlen, bis folgende Größenverteilung der Partikel erreicht ist :

Medianwert 1,9 μm
min. 99 % < 6 μm
min. 90 % < 3 μm.

Die Bestimmung der Größe der Partikel und deren Verteilung erfolgt in einem Partikelmeßgerät, z. B. nach Suspendierung in Isopropanol. Die Abpackung der Mikropartikel erfolgt in 5 ml-Vials zu jeweils 2 g.

C) Zur Herstellung von 5 ml des gebrauchsfertigen Ultraschall-Kontrastmittels wird der Inhalt eines Vials mit Trägerflüssigkeit (steriles Wasser für Injektionszwecke, A) mittels einer Injektionsspritze in das Vial mit Mikropartikel (B) gegeben und bis zum Entstehen einer homogenen Suspension geschüttelt (5 bis 10 Sekunden).

Beispiel 12

A) Herstellung der Trägerflüssigkeit
Wasser für Injektionszwecke wird zu jeweils 4 ml in 5 ml-Vials abgefüllt und 20 Minuten bei 120 °C sterilisiert.
B) Herstellung der Mikropartikel
Unter sterilen Bedingungen wird eine sterilfiltrierte Lösung von 0,5 g Saccharosedistearat in 40 g Isopropanol auf 199,5 g sterile Galactosepartikel aufgezogen, bei 40 °C und 200 Torr das Isopropanol abgetrocknet und mit einer Luftstrahlmühle vermahlen, bis folgende Größenverteilung der Partikel erreicht ist :

Medianwert 1,9 μm
min. 99 % < 6 μm
min. 90 % < 3 μm.

Die Bestimmung der Partikelgröße und deren Verteilung erfolgt in einem Partikelmeßgerät, z. B. nach Suspendierung in Isopropanol. Die Abpackung der Mikropartikel erfolgt in 5 ml-Vials zu jeweils 2 g.

C) Zur Herstellung von 5 ml des gebrauchsfertigen Ultraschall-Kontrastmittels wird der Inhalt eines Vials mit Trägerflüssigkeit (Wasser für Injektionszwecke, A) mittels einer Injektionsspritze in das Vial mit Mikropartikel (B) gegeben und bis zum Entstehen einer homogenen Suspension geschüttelt (5 bis 10 Sekunden).

Beispiel 13

A) Herstellung der Trägerflüssigkeit
Es wird Wasser für Injektionszwecke verwendet, jeweils zu 4 ml in 5 ml-Vials abgefüllt und diese 20 Minuten bei 120 °C sterilisiert.
B) Herstellung der Mikropartikel
Unter sterilen Bedingungen werden 0,5 g Saccharosedistearat mit 199,5 g Galactose angerieben, intensiv gemischt, durch ein 0,8 mm-Sieb gegeben und mit einer Luftstrahlmühle vermahlen, bis folgende Größenverteilung der Partikel erreicht ist :

Medianwert 1,9 μm
min. 99 % < 6 μm
min. 90 % < 3 μm.

Die Bestimmung der Größe der Partikel und deren Verteilung erfolgt in einem Partikelmeßgerät, z. B. nach Suspendierung in Isopropanol. Die Abpackung der Mikropartikel erfolgt in 5 ml-Vials zu jeweils 2 g.

C) Zur Herstellung von 5 ml des gebrauchsfertigen Ultraschall-Kontrastmittels wird der Inhalt eines Vials mit Trägerflüssigkeit (steriles Wasser für Injektionszwecke, A) mittels einer Injektionsspritze in das Vial mit Mikropartikel (B) gegeben und bis zum Entstehen einer homogenen Suspension geschüttelt (5 bis 10 Sekunden).

**Patentansprüche**

1. Mikropartikel und Gasbläschen enthaltende Kontrastmittel für die Ultraschall-Diagnostik, dadurch gekennzeichnet, daß es Mikropartikel einer festen, grenzflächenaktiven Substanz, gegebenenfalls in Kombination mit Mikropartikel eines nicht grenzflächenaktiven Feststoffes in einem flüssigen Träger enthält.

2. Mittel nach Anspruch 1, dadurch gekennzeichnet, daß es als feste grenzflächenaktive Substanz Lecithine, Polyoxyethylenfettsäureester, Glycerinpolyethylenglykolrhizinoleat, Cholesterol, Polyoxyethylenpolyoxypropylen-Polymere, Saccharoseester, Xyloglyceride, gesättigte oder ungesättigte (C₄-C₂₀)-Fettalkohole, gesättigte oder ungesättigte (C₄-C₂₀)-Fettsäuren oder deren Metallsalze, Mono-, Di- und Triglyceride, Fettsäureester als Mikropartikel in einer Menge von 0,01 bis 10 Gewichtsprozent enthält.

3. Mittel nach Anspruch 1 und 2, dadurch gekennzeichnet, daß es Magnesiumstearat, Ascorbylpalmitat, Saccharosemonopalmitat, Saccharosemonostearat oder Saccharosedistearat als feste grenzflächenaktive Substanz in Form von Mikropartikel in einer Konzentration von 0,01 bis 5 Gewichtsprozent, vorzugsweise 0,04 bis 1 Gewichtsprozent, enthält.

4. Mittel nach Anspruch 1, dadurch gekennzeichnet, daß es als gegebenenfalls vorhandene Mikropartikel eines nicht-grenzflächenaktiven Feststoffes Cyclodextrine, Monosaccharide, Disaccharide, Trisaccharide, Polyole oder anorganische oder organische Salze als Mikropartikel mit einer Konzentration von 5 bis 50 Gewichtsprozent enthält.

5. Mittel nach Anspruch 1 und 4, dadurch gekennzeichnet, daß es als gegebenenfalls vorhandenen nicht-grenzflächenaktiven Feststoff Galactose, Lactose oder α-Cyclodextrin als Mikropartikel in einer Konzentration von 5 bis 50 Gewichtsprozent, vorzugsweise von 9 bis 40 Gewichtsprozent, enthält.

6. Mittel nach Anspruch 1, dadurch gekennzeichnet, daß es als physiologisch verträglichen flüssigen Träger Wasser, physiologische Elektrolytlösung, die wässrige Lösung von ein- oder mehrwertigen Alkoholen wie Glycerin, Polyethylenglykol oder von Propylenglykolmethylester oder die wässrige Lösung eines Mono- oder Disaccharides enthält.

7. Mittel nach Anspruch 1 und 6, dadurch gekennzeichnet, daß es als physiologisch verträglichen flüssigen Träger Wasser, physiologische Kochsalzlösung, 10 %ige wässrige Lactose-Lösung oder 20 %ige wässrige Galactose-Lösung enthält.

8. Mittel nach Anspruch 1, dadurch gekennzeichnet, daß es Mikropartikel von Magnesiumstearat und von Galactose in einer 20 %igen wässrigen Galactose-Lösung enthält.

9. Mittel nach Anspruch 1, dadurch gekennzeichnet, daß es Mikropartikel von Ascorbylpalmitat und von Galactose in Wasser enthält.

10. Mittel nach Anspruch 1, dadurch gekennzeichnet, daß es Mikropartikel von Ascorbylpalmitat und von α-Cyclodextrin in physiologischer Kochsalzlösung enthält.

11. Mittel nach Anspruch 1, dadurch gekennzeichnet, daß es Mikropartikel von Ascorbylpalmitat in einer 10 %igen wäßrigen Lactoselösung enthält.

12. Mittel nach Anspruch 1, dadurch gekennzeichnet, daß es Mikropartikel von Saccharosemonopalmitat und von Galactose in Wasser enthält.

13. Mittel nach Anspruch 1, dadurch gekennzeichnet, daß es Mikropartikel von Saccharosemonostearat und von Galactose in Wasser enthält.

14. Mittel nach Anspruch 1, dadurch gekennzeichnet, daß es Mikropartikel von Saccharosedistearat und von Galactose in Wasser enthält.

15. Ein Kit für die Herstellung eines Mikropartikel und Gasbläschen enthaltenden Ultraschall-Kontrastmittels bestehend

a) aus einem Behälter mit einem Volumen von 5-10 ml, versehen mit einem Verschluß, der die Entnahme des Inhalts unter sterilen Bedingungen ermöglicht, gefüllt mit 4 ml des flüssigen Trägers und

b) aus einem zweiten Behälter mit einem Volumen von 5-10 ml, versehen mit einem Verschluß, der die Entnahme des Inhalts oder Zugabe eines Stoffgemisches unter sterilen Bedingungen ermöglicht, gefüllt mit Mikropartikel einer festen grenzflächenaktiven Substanz, gegebenenfalls in Kombination mit Mikropartikel eines nichtgrenzflächenaktiven Feststoffes mit einer durchschnittlichen Partikelgröße von < 1 bis 10 μm, wobei das Gewichtsverhältnis von grenzflächenaktiver Substanz zu gegebenenfalls vorhandenen nichtgrenzflächenaktivem Feststoff 0,01 bis 5 zu 100 beträgt und die Mikropartikel in einer Menge von 5 bis 50 Gewichtsprozent, vorzugsweise von 9 bis 40 Gewichtsprozent, enthalten sind.

16. Verfahren zur Herstellung eines Mikropartikel und Gasbläschen enthaltenden Kontrastmittels für die Ultraschall-Diagnostik, dadurch gekennzeichnet, daß man Mikropartikel einer physiologisch verträglichen, festen grenzflächenaktiven Substanz gegebenenfalls in Kombination mit Mikropartikel eines physiologisch verträglichen nicht-grenzflächenaktiven Feststoffes mit einer physiologisch verträglichen Trägerflüssigkeit vereinigt und bis zum Entstehen einer homogenen Suspension schüttelt.

**Claims**

1. Contrast medium containing microparticles and gas bubbles for ultrasound diagnostics, characterised in that it contains microparticles of a solid surface-active substance, optionally in combination with microparticles of a non-surface-active solid, in a liquid carrier.

2. Medium according to claim 1, characterised in that it contains as solid surface-active substance lecithins, polyoxyethylene fatty acid esters, glycerine polyethylene glycol ricinoleate, cholesterol, polyoxyethylenepolyoxypropylene polymers, saccharose esters, xyloglycerides, saturated or unsaturated $(C_4-C_{20})$-fatty alcohols, saturated or unsaturated $(C_4-C_{20})$-fatty acids or the metal salts thereof, mono-, di- and tri-glycerides or fatty acid esters, in the form of microparticles in a quantity of from 0.01 to 10 % by weight.

3. Medium according to claims 1 and 2, characterised in that it contains magnesium stearate, ascorbyl palmitate, saccharose monopalmitate, saccharose monostearate or saccharose distearate as solid surface-active substance in the form of microparticles in a concentration of from 0.01 to 5 % by weight, preferably from 0.04 to 1 % by weight.

4. Medium according to claim 1, characterised in that it contains as optionally present microparticles of a non-surface-active solid cyclodextrins, monosaccharides, disaccharides, trisaccharides, polyols or inorganic or organic salts in the form of microparticles in a concentration of from 5 to 50 % by weight.

5. Medium according to claims 1 and 4, characterised in that it contains as optionally present non-surface-active solid galactose, lactose or α-cyclodextrin in the form of microparticles in a concentration of from 5 to 50 % by weight, preferably from 9 to 40 % by weight.

6. Medium according to claim 1, characterised in that it contains as physiologically tolerable liquid carrier water, physiological electrolyte solution, an aqueous solution of monohydric or polyhydric alcohols, such as glycerine, polyethylene glycol or propylene glycol methyl ester, or an aqueous solution of a mono- or disaccharide.

7. Medium according to claims 1 and 6, characterised in that it contains as physiologically tolerable liquid carrier water, physiological saline, 10 % aqueous lactose solution or 20 % aqueous galactose solution.

8. Medium according to claim 1, characterised in that it contains microparticles of magnesium stearate and galactose in a 20 % aqueous galactose solution.

9. Medium according to claim 1, characterised in that it contains microparticles of ascorbyl palmitate and galactose in water.

10. Medium according to claim 1, characterised in that it contains microparticles of ascorbyl palmitate and α-cyclodextrin in physiological saline.

11. Medium according to claim 1, characterised in that it contains microparticles of ascorbyl palmitate in a 10 % aqueous lactose solution.

12. Medium according to claim 1, characterised in that it contains microparticles of saccharose monopalmitate and galactose in water.

13. Medium according to claim 1, characterised in that it contains microparticles of saccharose monostearate and galactose in water.

14. Medium according to claim 1, characterised in that it contains microparticles of saccharose distearate and galactose in water.

15. A kit for the preparation of an ultrasound contrast medium containing microparticles and gas bubbles, comprising

a) a container having a volume of from 5 to 10 ml which is provided with a closure permitting the removal of the contents under sterile conditions and which is filled with 4 ml, of the liquid carrier and

b) a second container having a volume of from 5 to 10 ml which is provided with a closure permitting the removal of the contents or the addition of a mixture of substances under sterile conditions and which is filled with microparticles of a solid surface-active substance, optionally in combination with micro-particles of a non-surface-active solid, having an average particle size of from < 1 to 10 μm, the ratio by weight of surface-active substance to optionally present non-surface-active solid being from 0.01 to 5 : 100 and the content of microparticles being from 5 to 50 % by weight, preferably from 9 to 40 % by weight.

16. Process for the preparation of a contrast medium containing microparticles and gas bubbles for ultrasound diagnostics, characterised in that microparticles of a physiologically tolerable solid surface-active substance, optionally in combination with micro-particles of a physiologically tolerable non-surface-active solid, are combined with a physiologically tolerable carrier liquid and shaken until a homogeneous suspension is formed.

**Revendications**

1. Produit de contraste contenant des microparticules et des bulles de gaz pour diagnostic échographique, caractérisé en ce qu'il contient des microparticules d'une substance tensioactive solide, éventuellement en combinaison avec des microparticules d'un solide non-tensioactif, dans un véhicule liquide.

2. Produit selon la revendication 1, caractérisé en ce qu'il contient en tant que substance tensioactive solide des lécithines, des esters d'acides gras de polyéthylèneglycol, du polyéthylèneglycol-ricinoléate de glycérol, du cholestérol, des polymères polyéthoxylés/polypropoxylés, des esters de saccharose, des xyloglycérides, des alcools gras en $C_4-C_{20}$ saturés ou insaturés, des acides gras en $C_4-C_{20}$ saturés ou insaturés ou leurs sels métalliques, des mono-, di- et triglycérides, des esters d'acides gras, sous la forme de microparticules en une quantité de 0,01 à 10 % en poids.

3. Produit selon les revendications 1 et 2, caractérisé en ce qu'il contient du stéarate de magnésium, du palmitate d'ascorbyle, du monopalmitate de saccharose, du monostéarate de saccharose ou du distéarate de saccharose en tant que substance tensioactive solide, sous forme de microparticules à une concentration de 0,01 à 5 % en poids, de préférence de 0,04 à 1 % en poids.

4. Produit selon la revendication 1, caractérisé en ce qu'il contient en tant que microparticules éventuellement présentes d'un solide non-tensioactif des cyclodextrines, monosaccharides, disaccharides, trisaccharides, polyols ou sels organiques ou inorganiques, sous forme de microparticules à une concentration de 5 à 50 % en poids.

5. Produit selon les revendications 1 et 4, caractérisé en ce qu'il contient en tant que solide non-tensioactif éventuellement présent du galactose, du lactose ou de l'α-cyclodextrine sous forme de microparticules à une concentration de 5 à 50 % en poids, de préférence de 9 à 40 % en poids.

6. Produit selon la revendication 1, caractérisé en ce qu'il contient en tant que véhicule liquide physiologiquement toléré l'eau, une solution physiologique d'électrolytes, une solution aqueuse de mono- ou polyalcools, comme le glycérol, le polyéthylèneglycol, ou de l'ester méthylique du propylèneglycol, ou encore une solution aqueuse d'un mono- ou d'un disaccharide.

7. Produit selon les revendications 1 et 6, caractérisé en ce qu'il contient en tant que véhicule liquide physiologiquement toléré de l'eau, une solution physiologique de chlorure de sodium, une solution aqueuse de lactose à 10 % ou une solution aqueuse de galactose à 20 %.

8. Produit selon la revendication 1, caractérisé en ce qu'il contient des particules de stéarate de magnésium et de galactose dans une solution aqueuse de galactose à 20 %.

9. Produit selon la revendication 1, caractérisé en ce qu'il contient des microparticules de palmitate d'ascorbyle et de galactose dans l'eau.

10. Produit selon la revendication 1, caractérisé en ce qu'il contient des microparticules de palmitate d'ascorbyle et d'α-cyclodextrine dans une solution physiologique de chlorure de sodium.

11. Produit selon la revendication 1, caractérisé en ce qu'il contient des microparticules de palmitate d'ascorbyle dans une solution aqueuse de lactose à 10 %.

12. Produit selon la revendication 1, caractérisé en ce qu'il contient des microparticules de monopalmitate de saccharose et de galactose dans l'eau.

13. Produit selon la revendication 1, caractérisé en ce qu'il contient des microparticules de monostéarate de saccharose et de galactose dans l'eau.

14. Produit selon la revendication 1, caractérisé en ce qu'il contient des microparticules de distéarate de saccharose et de galactose dans l'eau.

15. Trousse pour la préparation d'un produit de contraste échographique contenant des microparticules et des bulles de gaz, comprenant

a) un récipient ayant un volume de 5-10 ml, pourvu d'une fermeture permettant de prélever le contenu dans des conditions stériles, rempli de 4 ml du véhicule liquide, et

b) un deuxième récipient ayant un volume de 5-10 ml, pourvu d'une fermeture permettant de prélever le contenu ou d'ajouter un mélange de substances dans des conditions stériles, rempli de microparticules d'une substance tensioactive solide, éventuellement en combinaison avec des microparticules d'un solide non-tensioactif ayant une granulométrie moyenne comprise entre < 1 et 10 μm, le rapport pondéral entre la substance tensioactive et le solide non-tensioactif éventuellement présent étant compris dans l'intervalle 0,01-5 à 100, les microparticules étant contenues en une quantité de 5 à 50 % en poids, de préférence de 9 à 40 % en poids.

16. Procédé pour la préparation d'un produit de contraste contenant des microparticules et des bulles de gaz pour diagnostic échographique, caractérisé en ce qu'on réunit des microparticules d'une substance tensioactive solide physiologiquement tolérée, éventuellement en combinaison avec des microparticules d'un solide non-tensioactif physiologiquement toléré, et un liquide véhiculeur physiologiquement toléré, et qu'on secoue jusqu'à obtention d'une suspension homogène.